# EUROPEAN PATENT APPLICATION

(11) **EP 2 772 244 A1**
(43) Date of publication of application: **03.09.2014**
(21) Application number: 11874796.3
(22) Date of filing: 28.10.2011
(51) Int. Cl.: A61K 6/087, A61C 5/08, A61C 7/00, A61C 13/007, A61C 13/01, A61C 13/087, A61K 6/00

(54) **MOLDED BODY FOR DENTAL USE**

(71) Applicant: Nissin Dental Products Inc., Kyoto-shi, Kyoto 601-8469 (JP)
(72) Inventor: HISHIMOTO, Munemitsu, Kameoka-shi Kyoto 621-0001 (JP); KATO, Yoko, Kameoka-shi Kyoto 621-0001 (JP)
(74) Representative: Morf, Jan Stefan
(86) International application number: PCT/JP2011/074968
(87) International publication number: WO 2013/061462

(57) **Abstract**

Provided is a molded body for dental use, said molded body being made from a polyester resin which as a material for esthetic dentures exhibits excellent fracture resistance and moderate bendability, and which ensures excellent polishability during denture fabrication, and has an alkali resistance which prevents a denture from fracturing due to denture cleaning. This molded body for dental use is made from a polyester resin which is composed of a dicarboxylic acid component containing a terephthalic acid residue, and a glycol component containing a 2,2,4,4-tetramethyl-1, 3-cyclobutanediol residue and a 1, 4-cyclohexanedimethanol residue, wherein the proportions of the terephthalic acid residue, 2,2,4,4-tetramethyl-1,3-cyclobutanediol residue and 1,4-cyclohexanedimethanol residue in the polyester resin are 30 to 70 mol%, 5 to 25 mol%, and 25 to 50 mol%, respectively.

## Description

### TECHNICAL FIELD

The present invention relates to a molded body for dental use which has a moderate bendability and an excellent fracture resistance, polishability and alkali resistance, and can further satisfy various necessary properties required for an esthetic denture, a denture base, an artificial tooth, a dental temporary crown, a dental CAD/CAM block and prosthesis, a dental corrective device, and others.

### BACKGROUND ART

A primary purpose of denture is to improve the user's lost chewing function and restore the user's psychological state. However, conventional partial denture bases are poor in esthetic property, and this imposes a limit on restoring the user's psychological state. On the other hand, a partial denture base which does not have any metal clasp and in which a denture base is integrated with a clasp arm (= an esthetic denture) can be said to be a prosthesis effective not only for improving the user's function but also for restoring the user's psychological state.

In recent years, esthetic dentures having excellent esthetic characteristics without using any metal have been used widely. Properties required for such dentures include low elastic modulus resistant to breaking (moderate bendability), high heat resistance, and repairable using conventional denture-repairing material. In Japan, various materials such as polyamide resin and polycarbonate resin are used as esthetic denture material. Polyamide resin has a high fracture resistance; however the denture is difficult to repair. Also, since the resin itself has a low elasticity and is viscous, it requires time to polish the resin during the finishing phase of denture fabrication. Polycarbonate resin has a high elastic modulus, which from the point of denture design is difficult to adapt to various clinical cases. It also has the tendency to crack when washing using a denture cleaner.

Thus, in Patent Document 1 listed blow, as a molded body for dental use made of a resin more suitable than polyamide resin and polycarbonate resin, the Applicant has proposed a product molded using a copolymerized polyester resin composed of polyethylene terephthalate (PET) structural units, and poly-1,4-dimethylenecyclohexane terephthalate (PCT) structural units. The copolymerized polyester resin described in Patent Document 1 has advantages such as moderate elasticity, good moldability and the denture being repairable using a dental self-curing polymerization resin (repairing resin). However, this resin has a problem where it may fracture under excessive "bending". Additionally, this resin has a poor polishability. For example an esthetic denture fabricated using this above-mentioned copolymerized polyester resin was difficult to apply the final polish to and conduct mirror finish polishing. Furthermore, this copolymerized polyester resin has a lower alkali resistance than polyamide resin, therefore like the polycarbonate resin it has experienced cracking during denture cleaning.

As described above, a material good in overall balance is currently not yet known.

Apart from the above, as a polyester resin higher in impact strength and hydrolysis stability than polyester resin and polycarbonate resin, Patent Documents 2 and 3 listed below each disclose a resin containing a dicarboxylic acid component in specific proportion and a glycol component in a specific proportion, and having a specific inherent viscosity and glass transition temperature. The documents also disclose that this polyester resin is used to produce products such as films and bottles, and is used for a component of medical instruments. However, the application of such polyester resin for various molded bodies for dental use, such as an esthetic denture is not known.

### PRIOR ART DOCUMENTS

### PATENT DOCUMENT

Patent Document 1: JP-A-2003-12434
Patent Document 2: JP-T-2009-513800
Patent Document 3: JP-T-2009-513801

### SUMMARY OF THE INVENTION

### PROBLEMS TO BE SOLVED BY THE INVENTION

An objective of the present invention is to solve the above-mentioned problems in the prior art, and provide a molded body for dental use which has a moderate bendability and an excellent fracture resistance, polishability, and alkali resistance when washing the denture, and which can sufficiently satisfy various necessary properties required for an esthetic denture, a denture base, an artificial tooth, a dental temporary crown, a dental CAD/CAM block and prosthesis, and others.

A desired esthetic denture material has a flexible material quality which is capable of dealing with rotated teeth in a remaining dentition and various clinical cases from the viewpoint of denture design. The inventors have made detailed investigations about various alternative materials and as a result found out that: a polyester resin containing newly introduced substituents, that is, a polyester resin containing a phthalic acid residue, a 2,2,4,4-tetramethyl-1,3-cyclobutanediol residue, and a cyclohexanedimethanol residue sufficiently satisfies various properties required for an esthetic denture and other molded bodies for dental use (such as bendability, impact resistance, adhesive property, polishability, and alkali resistance); and is a molding material for dental use which causes no problem during actual use. Thus, the present invention has been achieved.

### MEANS FOR SOLVING THE PROBLEMS

The molded body for dental use of the present invention, which can solve the above-mentioned problems, features a molded body having a predetermined shape used in the oral cavity, and is made from a polyester resin composed of a dicarboxylic acid component containing a terephthalic acid (TPA) residue, and a glycol component containing a 2,2,4,4-tetramethyl-1,3-cyclobutanediol (TMCD) residue and a 1,4-cyclohexanedimethanol (CHDM) residue.

The present invention is also characterized by that, in the above-mentioned molded body for dental use, the proportion of the terephthalic acid residue in the polyester resin is from 30 to 70 mol%, that of the 2,2,4,4-tetramethyl-1,3-cyclobutanediol residue is from 5 to 25 mol%, and that of the 1,4-cyclohexanedimethanol residue is from 25 to 50 mol%.

The present invention is also characterized by that, in the above-mentioned molded body for dental use, the proportion of the terephthalic acid residue in the polyester resin is from 40 to 60 mol%, that of the 2,2,4,4-tetramethyl-1,3-cyclobutanediol residue is from 10 to 20 mol%, and that of the 1,4-cyclohexanedimethanol residue is from 30 to 40 mol%.

The present invention is also characterized by that, in the above-mentioned molded body for dental use, the polyester resin has a glass transition temperature Tg of 99 to 125°C.

Further, the present invention features the above-mentioned molded body for dental use having a form selected from the group consisting of an esthetic denture, a denture base, an artificial tooth, a dental temporary crown, a dental CAD/CAM block and prosthesis, and a dental corrective device.

### EFFECT OF THE INVENTION

The polyester resin constituting the molded body for dental use of the present invention comprises a dicarboxylic acid component containing a TPA residue, and a glycol component containing a TMCD residue and a CHDM residue. Therefore, this resin does not cause any problems related to fears that bisphenol A is eluted or generated when the resin is used, as seen in conventional polycarbonate resins. Thus, the molded body for dental use can be used safely as medical equipment and its safety is approved by the Japanese Food Sanitation Law and the FDA. Additionally, this polyester resin is high in mechanical properties, is bendable and resistant to breaking and satisfies all of the various above-mentioned requirements for an esthetic denture. Furthermore, the resin has good moldability and can be easily fabricated into various forms using an ordinary molding machine, such as an esthetic denture, a dental temporary crown, a denture base, and an artificial tooth.

Regarding the polyester resin which constitutes this molded body for dental use, the material itself is a colorless and transparent tone which allows the molded body to be colored into the desired color. Also, the resin does not crack easily due to its excellent impact resistance. Furthermore, the resin has good grindability and polishability and mirror-finish polishing can be applied with ease.

A denture base is exposed to use under harsh conditions in the oral cavity, may be damaged or break under use and experiences changes in the shape of the oral cavity over time. Due to these issues, a denture base is oftentimes repaired.

Thus, the denture base is required to have an adhesive property that can bond to any conventional dental repair resin (MMA-PMMA based resin). For an esthetic denture, polyamide resin, which has a high fracture resistance, is used in many cases. However, repairing becomes difficult since polyamide resin does not bond well to dental repair resin due to its poor adhesive property. Polycarbonate resin has a high adhesive strength; however, this resin may produce cracks when it contacts the dental repair resin, which may leave the repaired section noticeable.

By contrast, the polyester resin in this invention has excellent solvent resistance, and exhibits excellent adhesive property without developing surface roughness or fracture when it contacts the dental repair resin; thus, a good repair result can be achieved when this polyester resin is used.

### MODE FOR CARRYING OUT THE INVENTION

The molded body for dental use of the present invention is made from a polyester resin composed of a dicarboxylic acid component containing a TPA residue, and a glycol component containing a TMCD residue and a CHDM residue. This resin can be produced by polymerizing these components. In the present invention, it is preferred that: the proportion of the TPA residue in the polyester resin suited for constituting a molded body for dental use such as esthetic dentures, is from 30 to 70 mol%, preferably from 40 to 60 mol%, that of the TMCD residue is from 5 to 25 mol%, preferably from 10 to 20 mol%, and that of the CHDM residue is from 25 to 50 mol%, preferably from 30 to 40 mol%; and the glass transition temperature Tg of the resin ranges from 99 to 125°C. The polyester resin having such constituent components has the following characteristics, which is different from ordinary PET's: the resin becomes fracture resistant by the introduction of the TMCD residue; polishability is improved since the viscousness caused by softening of the resin surface by heat generation during polishing is decreased, therefore a mirror-polished surface can be easily achieved; and further the resin is reformed into an amorphous form by the introduction of the CHDM residue to give high transparency and mechanical properties in various molding methods.

The polyester resin comprising the above-mentioned constituent components can be produced by methods described in Patent Documents 2 and 3. However, commercially available resin can also be used.

A method for forming the molded body for dental use of the present invention by use of this polyester resin composition may be a known method such as injection molding method or compression molding method. The polyester resin, which constitutes the molded body for dental use of the invention, is very good in formability. Accordingly, general conditions can be applied for molding conditions in the individual molding methods. Thus, the resin can be easily fabricated using an ordinary molding machine.

The polyester resin, which constitutes the molded body for dental use of the present invention, is very useful in the field of dentistry as a raw material alternative for polycarbonate resin, and is particularly suitable for an esthetic denture, a dental temporary crown, a denture base, an artificial tooth, a dental corrective device (particularly, the molded plastic section of a dental orthodontic brace), and others. When the molded body for dental use needs to be improved in characteristics or mechanical property in the invention, inorganic filler, glass fiber, carbon fiber, or some other filler may be blended and filled thereinto. A colorant such as an inorganic pigment, an organic pigment or a dye may be added to the molded body for dental use of the invention. Aramid fiber, rayon fiber or the like may be added thereto as a material for revealing a simulated blood vessel.

Hereinafter, examples of the invention will be described; however, the invention is not limited thereto.

### EXAMPLES

### Example 1: Synthetic example of a polyester resin constituting a molded body for dental use of the present invention

A mixture of 77.7 g of dimethyl terephthalate, 48.5 g of 1,4-cyclohexanedimethanol, 17.9 g of 2,2,4,4-tetramethyl-1,3-cyclobutanediol, and 0.046 g of dibutyltin oxide were added into a 500-mL flask equipped with a nitrogen injecting port, a metal stirrer and a short distilling column. This flask was placed into a Wood' s metal bath preheated to 210°C. The mixing speed was set to 200 RPM throughout the entire experiment. The content in the flask was heated at 210°C for 5 minutes; then the temperature was gradually raised to 290°C over 30 minutes. The reaction mixture was maintained at 290°C for 60 minutes; then the pressure in the flask was gradually reduced over the next 5 minutes until the pressure reached 100 mmHg. The pressure in the flask was further reduced over the next 5 minutes to 0.3 mmHg. The 0.3 mmHg pressure was maintained for a total of 90 minutes to remove an excessive unreacted fraction of the diol. As a result, a polymer was obtained which was high in melt viscosity, and clear and colorless under visual observation. The glass transition temperature thereof was 101°C. An NMR analysis thereof demonstrated that the resultant polymer (polyester resin) was composed of 50 mol% of a terephthalic acid residue, 11 mol% of a 2,2,4,4-tetramethyl-1,3-cyclobutanediol residue, and 39 mol% of a 1,4-cyclohexanedimethanol residue.

### Example 2: Physical property comparative experiments

Experiments were conducted for comparing various physical properties between the polyester resin constituting a molded body for dental use of the present invention, and conventionally used resins (a polyamide resin, a polyester resin, and a polycarbonate resin). The resins used in the experiments were as follows:
Present invention product: polyester resin produced in Example 1
   Conventional product 1: polyamide resin (commercially available thermoplastic resin for denture bases)
   Conventional product 2: polyester resin (commercially available thermoplastic resin for denture bases)
   Conventional product 3: polycarbonate resin (commercially available thermoplastic resin for denture bases)

### 1. Bending strength · flexural modulus test

The resin of each of the present invention product and the conventional products 1-3 was used and formed into a test body in accordance with ISO 20795 (2008), and was tested using a universal testing machine (Autograph AG-1, manufactured by Shimadzu Corp.). The results are shown in Table 1 below. The bending strength and the bend elastic constant required for a denture base of an esthetic denture are about 60 MPa or more, and from about 1000 to 2000 MPa, respectively.

**[Table 1]**

| | Bending strength/MPa | Bend elastic constant/MPa | Judgment |
|---|---|---|---|
| Present invention product | 61.1 | 1497.8 | ○ |
| Conventional product 1 | 40.3 | 664.8 | × |
| Conventional product 2 | 73.4 | 1965.5 | Δ |
| Conventional product 3 | 88.0 | 2241.0 | × |

| | | | |
|---|---|---|---|
| Judgment criterion ○ Moderate bendability Δ Slightly difficult to bend × Excessive bending, or difficult to bend | | | |

### 2. Charpy impact test

The resin of each of the present invention product and the conventional products 1-3 was used and formed into a test body in accordance with ISO 1567 (1999). The test body was tested using a Charpy impact tester (impact tester DG-CB, manufactured by TOYO SEIKI SEISAKU-SHO, Ltd.). The results are shown in Table 2 below. The Charpy impact strength required for a material of an esthetic denture that does not break under excessive bending thereof is approximately 65 kJ/m² or more.

**[Table 2]**

| | kJ /m² | Judgment |
|---|---|---|
| Present invention product | 85.9 | ○ |
| Conventional product 1 | 81.7 | ○ |
| Conventional product 2 | 12.1 | × |
| Conventional product 3 | 65.0 | Δ |

| | | |
|---|---|---|
| Judgment criterion ○ High Δ Medium × Relatively low | | |

### 3. Adhesive property test

The resin of each of the present invention product and the conventional products 1~3 was used and formed into a test body of 15 x 15 x 3 mm in size. After adjusting the surface of each test body with sandpaper #240, a masking tape with a 5 mm diameter opening was affixed to define the bonding area. Next, while defining the bonding area with a 3mm thickness silicone frame with a 5 mm diameter opening, a dental self-curing resin MIKY PLUS (manufactured by Nissin Dental Products INC.) was filled and a pulling jig was planted on the test body. The test body was immersed in water at 37C for 24 hours; then the universal test machine was used to conduct a tensile test at a rate of 2 mm/min.

The adhesive property was verified by visually checking the finish of the bonding junction in addition to the bonding strength test. The results are shown in Table 3 below.

**[Table 3]**

| | Bonding strength/MPa | Adherend bonding condition | Appearance of bonding junction | Judgment |
|---|---|---|---|---|
| Present invention product | 14.3 | Adherend fracture | Bonding junction: unnoticeable | ○ |
| Conventional product 1 | 1.5 | Interfacial peeling | - | × |
| Conventional product 2 | 11.5 | Adherent fracture | Bonding junction: unnoticeable | ○ |
| Conventional product 3 | 15.9 | Mixed Fracture | Bonding junction: noticeable | Δ |

| | | | | |
|---|---|---|---|---|
| Judgment criterion ○ Good in strength and finish Δ Problem in either strength or finish × Bonding unsuccessful | | | | |

### 4. Polishability comparison test

A dental injection molding machine was used at each of six dental laboratories in the normal way for the injection molding of the resins of the present invention product and the conventional products 1-3, molded into dentures of similar shape. After removing the sprue from each of the molded bodies immediately after molding, a measurement was taken for the time required to conduct rough polishing to final polishing in the normal way. Relative comparison of the polishing time was conducted by presuming the polishing work time for the present invention product at 1.0. The results are shown in Table 4 below.

**[Table 4]**

| | Present invention product | Conventional product 1 | Conventional product 2 | Conventional product 3 |
|---|---|---|---|---|
| Dental laboratory A | 40 | 70 | 60 | 50 |
| Dental laboratory B | 20 | 60 | 60 | 60 |
| Dental laboratory C | 20 | 50 | 30 | 30 |
| Dental laboratory D | 30 | 60 | 50 | 40 |
| Dental laboratory E | 20 | 50 | 40 | 30 |
| Dental laboratory F | 20 | 60 | 40 | 40 |
| | | | | Unit: minute |

| | Present invention product | Conventional product 1 | Conventional product 2 | Conventional product 3 |
|---|---|---|---|---|
| Dental laboratory A | 1.0 | 1.8 | 1.5 | 1.3 |
| Dental laboratory B | 1.0 | 3.0 | 3.0 | 3.0 |
| Dental laboratory C | 1.0 | 2.5 | 1.5 | 1.5 |
| Dental laboratory D | 1.0 | 2.0 | 1.7 | 1.3 |
| Dental laboratory E | 1.0 | 2.5 | 2.0 | 1.5 |
| Dental laboratory F | 1.0 | 2.5 | 2.0 | 1.5 |
| Judgment | ○ | × | Δ | Δ |

| | | | | |
|---|---|---|---|---|
| Judgment criterion ○ Polishing time: short Δ Polishing time: medium × Polishing time: long | | | | |

### 5. Alkali resistance test

The resin of each of the products was subjected to a constant-strain solvent crack test while immersed in an aqueous alkali solution. In this test, each pre-formed molded body (a water-absorption · dissolution quantity test body according to ISO 20795 (2008) was used for this test) with a diameter of 50 mm and a thickness of 0.5 mm with both circular ends warped to become 45 mm in length was fixed into this warped state with a jig fixture. Then, the molded bodies were placed into glass vessels and immersed in aqueous sodium hydroxide solutions previously adjusted to have concentrations of 0.1 mol/L (pH: 13), 0.01 mol/L (pH: 12), 0.001 mol/L (pH: 11), and 0.0001 mol/L (pH: 10) at 23°C for one week. The molded bodies were then checked for any formation of fractures.

Further, various commercially available alkaline-based denture cleaners were tested in accordance with instructions, and the molded bodies were checked for formation of fractures in the same way as described above. The results are shown in Table 5 below.

**[Table 5]**

| | | Present invention product | Conventional product 1 | Conventional product 2 | Conventional product 3 |
|---|---|---|---|---|---|
| 0.1 mol/L NaOHaq | pH 13 | ○ | ○ | × | × |
| 0.01 mol/L NaOHaq | pH 12 | ○ | ○ | × | × |
| 0.001 mol/L NaOHaq | pH 11 | ○ | ○ | ○ | × |
| 0.0001 mol/L NaOHaq | pH 10 | ○ | ○ | ○ | ○ |
| Judgment | | ○ | ○ | × | × |
| | | | | | |

| Product name | | Present invention product | Conventional product 1 | Conventional product 2 | Conventional product 3 |
|---|---|---|---|---|---|
| Denture cleaner A | pH 12 | ○ | ○ | × | × |
| Denture cleaner B | pH 12 | ○ | ○ | × | × |
| Denture cleaner C | pH 9 | ○ | ○ | ○ | ○ |
| Denture cleaner D | pH 8 | ○ | ○ | ○ | ○ |
| Judgment | | ○ | ○ | × | × |

| | | | | | |
|---|---|---|---|---|---|
| Judgment ○ Does not fracture/good criterion × Fractures/bad | | | | | |

### 6. Overall evaluation · judgment

According to the above-mentioned test results, the present invention product had a moderate bending elasticity (moderate bendability) and a moderate impact resistance, and also had good adhesiveness with self-curing resin. In the case of conventional products 1~3, the required polishing time was about 1/2 to 1/3. It was also verified that the present invention product possesses an alkali resistance therefore does not fracture under use with denture cleaning agents. By contrast, it was verified that conventional product 1 was low in bending strength and flexural modulus and showed excessive bending, and had poor adhesiveness with self-curing resin, and poor polishability. Conventional product 2 had poor impact resistance and polishability, and fracturing caused by denture cleaning agents was observed. Conventional product 3 had a high flexural modulus and was difficult to bend, was poor in polishability, and fracturing caused by denture cleaning agents was observed.

### Example 3: Concrete example (1) of the molded body for dental use of the present invention: esthetic denture

A mixture was prepared which was obtained by mixing, with 100 parts by weight of the polyester resin produced in Example 1, 0.015 parts by weight of titanium oxide, 0.0005 parts by weight of solvent red 151, 0.002 parts by weight of red iron oxide and 0.001 parts by weight of black iron oxide as colorants. This mixture was used to fabricate a denture base of an esthetic denture formed by a dental injection molding machine.

It was verified that the thus fabricated denture base has a moderate bendability resistant to breaking, and has an excellent impact resistance; and when it was fitted and used in the patient no problem resulted whatsoever.

### Example 4: Concrete example (2) of the molded body for dental use of the present invention: dental CAD/CAM block and prosthesis

A mixture was prepared which was obtained by mixing, with 100 parts by weight of the polyester resin produced in Example 1, 0.03 parts by weight of titanium oxide, 0.03 parts by weight of titanium yellow and 0.001 parts by weight of red iron oxide as colorants. This mixture was molded into a block form by an extruder. Thereafter, a dental CAD/CAM machine was used to cut and mill the block into veneer crown form prosthesis.

At production, the block was able to be cut and milled by a dental CAD/CAM machine without any problem whatsoever. It was also verified that when the thus fabricated prosthesis was fitted and used in the patient, the prosthesis did not fracture and no problem resulted whatsoever.

### INDUSTRIAL APPLICABILITY

The polyester resin constituting the molded body for dental use of the present invention is excellent in fracture resistance, possesses a moderate amount of "bendability", has excellent polishability during denture fabrication, and good moldability. Thus, the resin is workable into various molded bodies for dental use. Particularly useful is a molded body that has been fabricated into an esthetic denture.

## Claims

1. A molded body for dental use having a predetermined shape used in an oral cavity, which is made from a polyester resin composed of a dicarboxylic acid component containing a terephthalic acid residue, and a glycol component containing a 2,2,4,4-tetramethyl-1,3-cyclobutanediol residue and a 1,4-cyclohexanedimethanol residue.

2. The molded body for dental use as defined in Claim 1, in which the proportion of the terephthalic acid residue in the polyester resin is from 30 to 70 mol%, that of the 2,2,4,4-tetramethyl-1,3-cyclobutanediol residue is from 5 to 25 mol%, and that of the 1,4-cyclohexanedimethanol residue is from 25 to 50 mol%.

3. The molded body for dental use as defined in Claim 1 or 2, in which the proportion of the terephthalic acid residue in the polyester resin is from 40 to 60 mol%, that of the 2,2,4,4-tetramethyl-1,3-cyclobutanediol residue is from 10 to 20 mol%, and that of the 1,4-cyclohexanedimethanol residue is from 30 to 40 mol%.

4. The molded body for dental use as defined in any one of Claims 1 to 3, in which the polyester resin has a glass transition temperature Tg of 99 to 125°C.

5. The molded body for dental use as defined in any one of Claims 1 to 4, which has a form selected from the group consisting of an esthetic denture, a denture base, an artificial tooth, a dental temporary crown, a dental CAD/CAM block and prosthesis, and a dental corrective device.
